# EUROPEAN PATENT APPLICATION

(11) **EP 4 843 978 A2**
(43) Date of publication of application: **25.11.2026**
(21) Application number: 26196457.1
(22) Date of filing: 23.11.2015
(51) Int. Cl.: C07C 211/29

(54) **NEW METHOD FOR SYNTHESIS OF FENFLURAMINE, OF ISOMERS THEREOF AND/OR OF ANALOGS THEREOF, AND NEW COMPOSITIONS COMPRISING IT**

(62) Document number(s) of the earlier application(s) in accordance with Art. 76 EPC: 15425100.3 / 3 170 807
(71) Applicant: Frau Pharma S.r.l., 20864 Agrate Brianza (MB) (IT)
(72) Inventor: PALOMBI, Giovanni, 20900 Monza (IT); ZAGAMI, Michael, 20023 Cerro Maggiore MI (IT)
(74) Representative: Modiano, Micaela Nadia

(57) **Abstract**

A new process for preparing the fenfluramine molecule or isomers thereof and/or analogs thereof and new compositions containing fenfluramine or isomers thereof and/or analogs thereof obtainable with the claimed process.

## Description

### Field of the invention

The present invention relates to a new, efficient and easily industrializable process for preparing the fenfluramine molecule, isomers thereof and/or analogs thereof, and at the same time relates to new compositions that contain fenfluramine, or isomers thereof and/or analogs thereof obtained with the process.

### Background art

Fenfluramine, i.e., 3-trifluoromethyl-N-ethylamphetamine, has the following chemical structure:

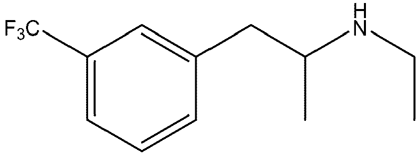

The marketing of fenfluramine as a pharmaceutical active ingredient in the United States began in 1973 and was used in a therapy in combination with phentermine to prevent and treat obesity. However, in 1997 fenfluramine was withdrawn from the market in the United States and immediately thereafter in other countries, since its ingestion was associated with the onset of cardiac fibrosis and pulmonary hypertension. As a consequence of this event, the pharmaceutical compounds containing this active ingredient were withdrawn from the market. However, fenfluramine, even after its exit from the market, has continued to attract scientific interest, as will become apparent from the discussion presented hereinafter.

In the literature, over the years, numerous syntheses or processes have been reported for preparing fenfluramine or its dextrorotatory enantiomer dexfenfluramine or an analog containing a highly electron-attractor group on the aromatic ring as in the fenfluramine molecule (see for example Pentafluorosulfanyl Serotonin Analogs: Synthesis, Characterization, and Biological Activity, John T. Welch and Dongsung Lim Chapter 8, pp 165-181 DOI: 10.1021/bk-2009-1003.ch008). Many of these synthesis paths are long and foresee multiple stages or synthesis steps that can include reagents that are dangerous or scarcely environment-friendly and are therefore scarcely convenient for an industrial synthesis. Hereinafter, any reference to "fenfluramine" is understood to refer to the racemic form, i.e, (RS)-N-ethyl-1-[3-(trifluoromethyl)phenyl]propan-2-amine.

To the best of the knowledge of the inventors, the first method for fenfluramine synthesis reported in the literature dates back to 1962 and is referenced in patent BE609630 and in analogous patents US3198833 and FR1324220. All the synthesis methods reported in these patents provide for numerous synthesis steps. By way of example, one of the methods provides for the transformation into oxime of a ketone, 1-(3-trifluoromethyl)phenyl-propan-2-one, as shown here:

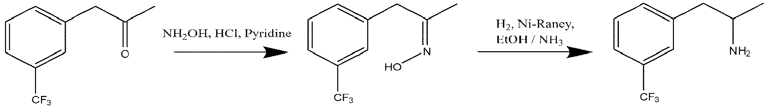

The oxime is then hydrogenated in the presence of Raney nickel catalyst so as to yield the corresponding primary amine, which is acetylated subsequently with ethanoic anhydride before being converted into fenfluramine by reduction with lithium aluminum hydride.

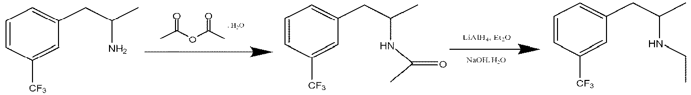

As can be seen, the final step of this chemical process provides for the use of lithium aluminum hydride and the persons skilled in the art will acknowledge that the use of this reagent should be avoided, if possible, on an industrial level, since it is extremely flammable and is the source of accidents. Furthermore, lithium is a potentially neurotoxic metal and therefore its use should be avoided where possible. Furthermore, the Raney nickel catalyst is used in the oxime reduction step and can contaminate the final active ingredient; the use of hydroxylamine also entails problems of toxicity for workers assigned to production.

A further disadvantage of this process is, as already mentioned earlier, the number of steps, not only because a large number of synthesis steps entails a reduction of the overall yield of active ingredient, but also because each synthesis step in principle can generate impurities and a larger number of steps can therefore entail a higher number of impurities in the final active ingredient. Many of these impurities, furthermore, due to their structural similarity to fenfluramine, are difficult to eliminate and remove from a fenfluramine preparation. One impurity for example that can be formed in the process described above and is difficult to eliminate is the following:

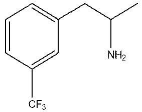

This impurity, which is a primary amine, shares physical-chemical properties that are similar to fenfluramine and therefore, like fenfluramine, it can form a hydrochloride salt by treatment with hydrochloric acid and thus contaminate the active ingredient fenfluramine hydrochloride. Furthermore, this impurity - as a free base - has a boiling point that is similar to that of fenfluramine (73°C vs. 89°C at 6 mmHg respectively), and therefore its elimination by distillation also can be problematic.

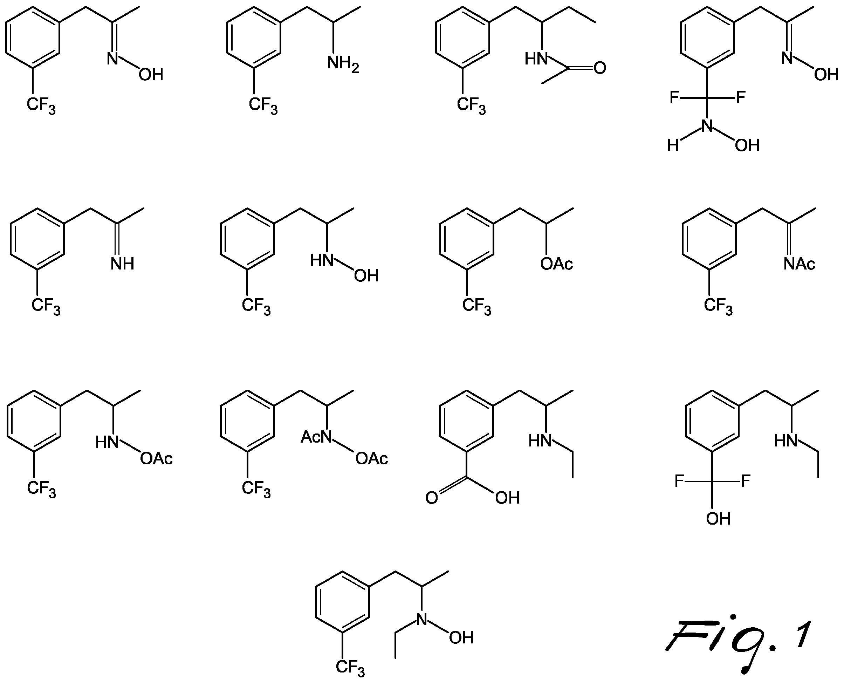

The process described above can in principle generate other impurities, which are listed in **Figure 1**.

EP 0441160 claims a synthesis in 5 steps of dexfenfluramine, dextrorotatory enantiomer of fenfluramine. This synthesis can be adapted easily to produce fenfluramine instead of its dextrorotatory enantiomer simply by performing the first reduction step with a non-chiral reducing agent. In the first step, in fact: 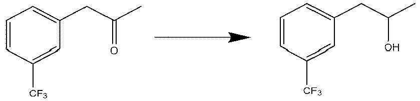 a ketone, 1-(3-trifluoromethyl)phenyl-propan-2-one, is first reduced to the corresponding alcohol in the presence of yeast, D-glucose, ethanol and water. Then the alcohol is converted into the tosylate in the second step:

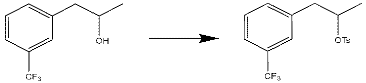

This reaction occurs in the presence of triethylamine and tosyl chloride in methylene chloride as solvent. After purification, the tosylate is converted to fenfluramine by means of three successive steps:

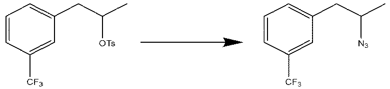

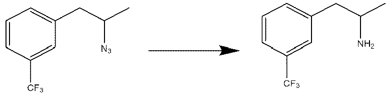

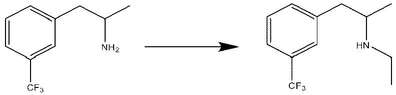

In the first of these three steps, the tosylate is converted into an azide intermediate by reaction with sodium azide in dimethylformamide. The azide intermediate is then hydrogenated in the presence of a catalyst, palladium on carbon. Finally, the resulting primary amine is converted into fenfluramine by reaction with acetaldehyde and sodium borohydride.

Persons skilled in the art may see easily that this process is not desirable from an industrial standpoint due to reasons related to environmental risk, safety and costs. For example, the sodium azide used in the process is a notoriously explosive compound and its use at the industrial level is dangerous. Furthermore, palladium is an expensive material and its use in the process entails an increase in the production costs of fenfluramine. Furthermore, palladium can contaminate the finished active ingredient.

In another method for the synthesis of dexfenfluramine in 3-4 steps, reported by Goument et al. in Bulletin of the Chemical Society of France (1993), 130, p. 450-458, 3-bromobenzotrifluoride is subjected to a Grignard reaction with enantiopure 1,2-propylene-epoxide to yield 1-[3-(trifluoromethyl)phenyl]propan-2-ol as shown hereafter:

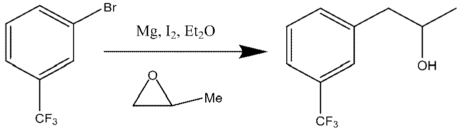

If this reaction is performed with racemic 1,2-propylene-epoxide, the synthesis can be adapted to the preparation of fenfluramine.

The alcohol thus obtained is first transformed into trifluoromethyl sulfonate by reaction with trifluoromethanesulfonic anhydride and then treated with ethylamine to yield fenfluramine, as shown in the diagram hereinafter:

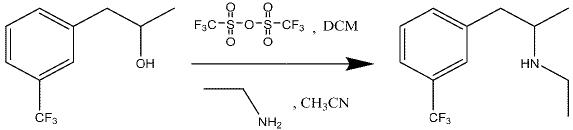

In this article, the authors acknowledge that the main byproducts of the reaction are isomer alkenes having the following chemical structures:

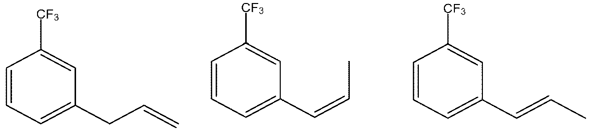

The process proposed by Goument et al. is not interesting from the industrial standpoint for a series of reasons. First of all, it is known that the use of Grignard reagents, especially on an industrial scale, is problematic, because these compounds are often pyrophoric and corrosive. Furthermore, 1,2-propylene epoxide is a suspected carcinogenic compound. Finally, the formation of the three isomer alkenes as byproducts listed above is a disadvantage of the process. In the article, Goument presents methods for activation of the intermediate alcohol which are alternative to trifluoromethylsulfonate, for example by converting it to chloride (via thionyl chloride) or to mesylate (via mesyl chloride), but these process variations share the same disadvantages as the main process analyzed above.

In addition to the methods with multiple synthesis steps discussed so far in detail, the literature reports other methods or processes for producing fenfluramine or dexfenfluramine. In general, persons skilled in the art acknowledge that the syntheses in the literature for producing dexfenfluramine sometimes can be applied to the preparation of fenfluramine simply by replacing the initial materials and/or enantiopure reagents with the corresponding racemates while maintaining the reaction conditions. For example, patents that present long synthesis methods in multiple steps are the following:
- DE1593595 and US3769319
- NL7215548
- EP810195 and EP882700 (dexfenfluramine)
- EP0301925 (dexfenfluramine)

Other examples of preparation of fenfluramine, taken from non-patent literature, are the following:
- Synthesis, Nov.1987, p. 1005-1007
- J.Org.Chem, 1991, 56, p. 6019
- Tetrahedron, 1994, 50(1), p. 171
- Bull. Soc. Chim. France, 1993, 130(4), p. 459-466 (dexfenfluramine)
- Chirality, 2002, 14(4), p. 325-328 (dexfenfluramine)

Without analyzing in detail the individual methods described in these patents or articles, it can be stated in summary that all these methods are not attractive and interesting from the industrial standpoint because these are processes with many synthesis steps or because the initial materials described therein are not easily available and therefore have to be prepared separately, with a further expenditure of time and with further costs, or because they provide for the use of reagents that are dangerous/explosive/toxic or because they entail the use of catalysts based on heavy metals that can contaminate the final active ingredient.

One should consider that in the literature there are methods for the preparation of fenfluramine that do not provide for long syntheses and multiple steps but are shorter and consist of one or two steps. These processes, which therefore would be more interesting from the industrial standpoint, have other specific disadvantages, as will become apparent in detail hereinafter. For example, in the literature there is a first group of articles or patents that describe the reaction between 1-(3-trifluoromethyl)phenyl-propan-2-one and ethylamine in the presence of hydrogen gas and of a transition metal as catalyst:

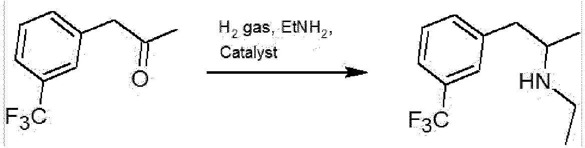

In particular, in Huagong Shikan, 2002, 16(7), p. 33, the reaction is performed with hydrogen gas (2.9 - 3.38 atm), at 65-75°C, for 9 hours, in the presence of Raney nickel. Likewise, in patent DD108971 (1973), Raney nickel and hydrogen gas and methanol are used as solvent to perform this reaction.

In HU55343, instead, a similar reaction in one step is performed with hydrogen gas in the presence of another transition metal catalyst, such as palladium on carbon.

Although these three methods describe short single-step processes, they have the disadvantage of the use of hydrogen gas. As is known to persons skilled in the art, hydrogen gas is a dangerous gas due to the inherent danger of forming explosive mixtures with air and must be used by expert personnel in expensive facilities dedicated to its use and built with special precautions. Despite being used in purpose-built facilities, the use of hydrogen at the industrial level is inherently dangerous and to be avoided if possible. Another danger element that is shared by the processes described above is the fact that the reactions are performed under pressure. The third industrial disadvantage then arises from the use of heavy metal catalysts, which have a high cost and therefore increase the overall cost of the final active ingredient and -on the other hand- may contaminate the active ingredient fenfluramine even after filtration of the catalyst and purification of said active ingredient.

Analysis of the background art shows, however, that an attempt has been made to devise a process for the production or synthesis of fenfluramine that is short (one or two steps) and does not entail the use of hydrogen gas or of catalysts based on nickel or palladium or the like. In particular, for example, Synthesis 1987, 11, p. 1005, and then DECHEMA Monographien (1989), 112 (Org. Elektrochem.--Angew. Elektrothermie), 367-74, present a method for the synthesis of fenfluramine which starts from 1-(3-trifluoromethyl)phenyl-propan-2-one, which is made to react with ethylamine in great excess, in an electrochemical process, which uses a mercury cathode in a water/ethanol solution with pH 10-11. One obtains fenfluramine with 87% yield. This process has some drawbacks from an industrial standpoint: it is a process of the electrochemical type and therefore requires special equipment which is scarcely widespread, dedicated cells and reactors, and it is not possible to use the classic multipurpose reactors available in the pharmaceutical industry. Furthermore, the use of mercury at the industrial level poses severe environment safety problems, requiring constant health monitoring on workers who manage the equipment and systems for the management and destruction of wastewater that are particularly onerous; finally, mercury can be transferred from the cathode to the reaction environment and therefore to the active ingredient, and this obviously is to be considered very dangerous due to the accumulation of the metal in human beings; small traces of mercury are very toxic.

Another method for fenfluramine synthesis in a single step is the one presented in J.Org.Chem, 1979, 44(20), p. 3580. Here the reaction is described between an alkene derivative and ethylamine in the presence of sodium borohydride and mercury nitrate:

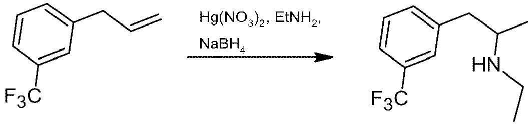

Again, this process is not interesting from an industrial standpoint since it has the same problems, if not even greater ones, related to the use of mercury (used here as a water-soluble salt) discussed previously. The complication introduced in this process with the use of mercury nitrate together with sodium borohydride highlights the level of innovation of the synthesis path found here.

In past years, therefore, it has not been possible to provide a process for synthesizing fenfluramine in a small number of steps by using modern reducing agents that are commonly and easily used. Indeed, while Gaodeng Xuexiao Huaxue Xuebao, 9(2), 1988, p. 134-139, describes and exemplifies the synthesis of 2-N-ethyl-1-phenyl propane by means of (1) the treatment of the precursor ketone with ethylamine followed by (2) sodium cyanoborohydride as reducing agent, Xuexiao Huaxue Xuebao provides no example for fenfluramine. Moreover, for the latter, Xuexiao Huaxue Xuebao indicates a melting point for the hydrochloride of 161°C, a data item that matches the value indicated in the literature initially (see BE609630); these facts prove that fenfluramine synthesis with cyanoborohydride was not performed, otherwise one cannot explain why the author did not transcribe, in the document, the example of a product that at the time was very important. It should be noted in fact that 1-phenyl propan-2-one and 1-(3-trifluoromethyl)phenyl-propan-2-one can have different reactivities to reductive amination due to the presence of a highly electron-attractor -trifluoromethyl group, hence the need for an example to demonstrate its feasibility. The use of cyanoborohydride shares some disadvantages with other methods discussed in the preceding paragraphs. The excellent selectivity for reductive aminations of this reagent is highly appreciated, but its application can be less advantageous with respect to other reducing systems in the synthesis of fenfluramine, where the latter is intended for therapeutic application in human beings. The reasons for this are the possible contamination of the finished pharmaceutical active ingredient with cyanide ions, the toxicity of the reagent itself and finally the danger of its use. It is known to persons skilled in the art that sodium cyanoborohydride can release hydrocyanic acid if the pH of the reaction environment is acid enough and it is known that hydrocyanic acid is a powerful poison, since it competes with oxygen for hemoglobin coordination. As a consequence of this, particular care must be taken in its use and in the disposal of the production wastewater, which can be contaminated by cyanides. Not least, one must consider that the cost of sodium cyanoborohydride is considerable.

To conclude, it can be seen that more than 50 years after the publication of its first synthesis dated 1962, there are still numerous disadvantages or limitations in the synthesis paths developed in the past decades in the literature for the preparation of fenfluramine.

Moreover, recently there has been renewed pharmaceutical interest in the fenfluramine molecule, since the possibility of its therapeutic use in severe disorders of infancy has appeared in the medical literature. For example, mention can made of Ceulemans et al., Epilepsia, 53(7), pages 1131 to 1139, 2012.

According to a certain part of medical literature, fenfluramine might therefore be interesting as a medication in a chronic therapy for the treatment of symptoms of epilepsy and other correlated severe disorders.

Based on recent medical developments, therefore, the need exists for a synthesis method that is better than the existing ones and can overcome in particular the disadvantages of the processes that are present in the literature. Particularly important, in view of use in chronic therapies for children such as epilepsy and other severe disorders, it would be fundamentally important to identify a path for synthesis of the active ingredient fenfluramine or of isomers thereof and/or analogs thereof that does not entail the use of heavy metals and/or transition metals, which in a chronic therapy might accumulate in the body of the patients over the years, with severe consequences on health.

More generally, it is desirable to identify a synthesis path that uses reagents from which (or from the transformation products of which) it is then possible to easily purify fenfluramine (or isomers and/or analogs thereof).

It would be equally desirable to identify a synthesis path that comprises a small number of synthesis steps and uses reagents that are widely commercially available and easy to use.

At the same time, the new identified synthesis path should avoid if possible the formation of byproducts.

### Description of the figures

**Figure 1****:** impurities generated theoretically by means of the reagents used in the first fenfluramine synthesis according to BE609630.

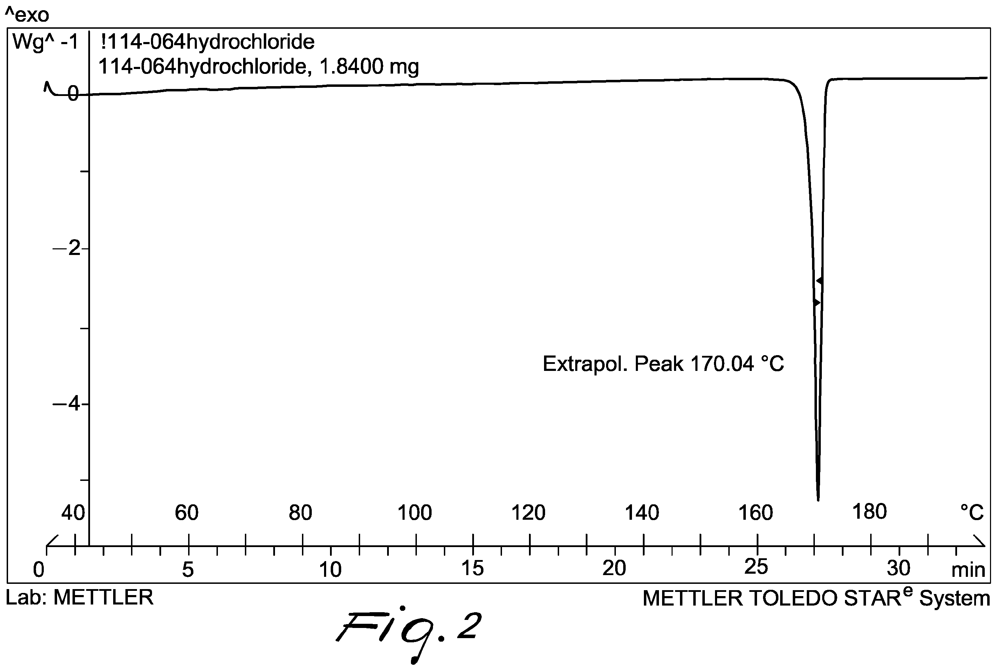

**Figure 2****:** DSC of crude fenfluramine hydrochloride, obtained by reduction with sodium cyanoborohydride according to test 16 (table **B**) of the description that follows.
**Figure 3****:** DSC of fenfluramine hydrochloride recrystallized from 2-butanol as in example 2b (reduction with sodium borohydride).

### Summary of the invention

The inventors of the present application have found surprisingly that the aim and objects indicated above are achieved by a new method for the synthesis of (RS)-N-ethyl-[phenyl]propan-2-amine substituted with R₁ on the phenyl ring or of a pharmaceutically acceptable salt thereof, comprising the transformation of a ketone having the structure (I): 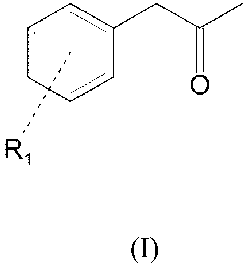
wherein R₁ is an electron-attractor group chosen in turn from the group that consists of CF₃, halogen, -SF₅, carboxyl group, trihalogen methyl group other than CF₃, mesylate, tosylate, triflate, nitro, phosphate, sulfonate and amide,
with ethylamine
and with a reducing agent chosen from the group consisting of alkaline cation or ammonium borohydride, alkaline cation or ammonium triacetoxyborohydride and alkaline cation or ammonium cyanoborohydride, in which the alkaline cation is always different from lithium cation and mixtures thereof, to yield (RS)-N-ethyl-[phenyl]propan-2-amine substituted with R₁ on the phenyl ring, optionally followed by the transformation of the obtained (RS)-N-ethyl-[phenyl]propan-2-amine substituted with R₁ on the phenyl ring into a pharmaceutically acceptable salt.

The method of the invention is preferred for the synthesis of fenfluramine or of an analog thereof as indicated hereinafter or of a pharmaceutically acceptable salt thereof, comprising the transformation of a ketone having the structure (II): 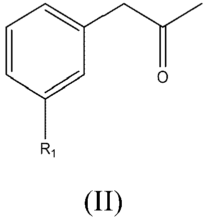
wherein R₁ is CF₃ or wherein R₁ is an electron-attractor group chosen in turn from the group that consists of halogen, -SF₅, carboxylic group, trihalogen methyl group other than CF₃, mesylate, tosylate, triflate, nitro, phosphate, sulfonate, and amide,
with ethylamine
and with a reducing agent chosen from the group consisting of alkaline cation or ammonium borohydride, alkaline cation or ammonium triacetoxyborohydride and alkaline cation or ammonium cyanoborohydride, in which the alkaline cation is always different from lithium cation and mixtures thereof, to yield fenfluramine or an analog thereof, in which R₁ is an electron-attractor group as above, optionally followed by the transformation of the obtained fenfluramine or of an analog thereof as above into a pharmaceutically acceptable salt.

Furthermore, the inventors of the present invention have also discovered a new preparation of fenfluramine, or of isomers and/or analogs thereof, which can be obtained by means of the method described hereinafter, and new pharmaceutical compositions that contain it.

### Detailed description of the invention

As mentioned above, it has been found that the aim and objects of the invention, and additional objects that will become better apparent hereinafter, can be achieved with a new method for the synthesis of fenfluramine or isomers thereof in which the CF₃ group can be present in any one of the positions on the phenyl ring and/or of analogs thereof in which the CF₃ group can be replaced with another electron-attractor substituent chosen from the group consisting of halogen, -SF₅, carboxylic group, trihalogen methyl group other than CF₃, mesylate, tosylate, triflate, nitro, phosphate, sulfonate, and amide.

In particular, the present invention provides the method according to the first claim, i.e., a method for the synthesis of (RS)-N-ethyl-[phenyl]propan-2-amine substituted with R₁ on the phenyl ring or of a pharmaceutically acceptable salt thereof, comprising the transformation of a ketone having the structure (I): 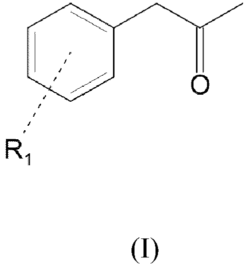
wherein R₁ is an electron-attractor group chosen in turn from the group consisting of CF₃, halogen, -SF₅, carboxylic group, trihalogen methyl group other than CF₃, mesylate, tosylate, triflate, nitro, phosphate, sulfonate, and amide,
with ethylamine
and with a reducing agent chosen from the group consisting of alkaline cation or ammonium borohydride, alkaline cation or ammonium triacetoxyborohydride and alkaline cation or ammonium cyanoborohydride, in which the alkaline cation is always different from lithium cation and mixtures thereof, to yield (RS)-N-ethyl-[phenyl]propan-2-amine substituted with R₁ on the phenyl ring, optionally followed by the transformation of the obtained (RS)-N-ethyl-[phenyl]propan-2-amine substituted with R₁ on the phenyl ring into a pharmaceutically acceptable salt. As indicated above, the cations of the reducing agent are ammonium or cations of alkaline metals other than lithium; among these, particular preference is given to sodium cation and potassium cation; more particularly, sodium cation is preferred.

The method according to the invention is preferred for the synthesis of fenfluramine or of an analog thereof as indicated hereinafter or of a pharmaceutically acceptable salt thereof, comprising the transformation of a ketone having the structure (II): 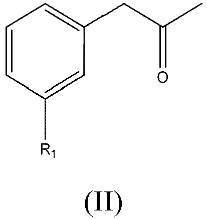
wherein R₁ is CF₃ or wherein R₁ is an electron-attractor group chosen in turn from the group consisting of halogen, -SF₅, carboxylic group, trihalogen methyl group other than CF₃, mesylate, tosylate, triflate, nitro, phosphate, sulfonate, and amide,
with ethylamine
and with a reducing agent chosen from the group consisting of alkaline cation or ammonium borohydride, alkaline cation or ammonium triacetoxyborohydride and alkaline cation or ammonium cyanoborohydride, in which the alkaline cation is always different from lithium cation and mixtures thereof,
to yield fenfluramine or an analog thereof, in which R₁ is an electron-attractor group as above, optionally followed by the transformation of the obtained fenfluramine or of an analog thereof as above into a pharmaceutically acceptable salt. As indicated above, the cations of the reducing agent are ammonium or cations of alkaline metals other than lithium; among these, particular preference is given to sodium cation and potassium cation; more particularly, sodium cation is preferred.

In the new process as above, the transformation of the ketone having the structure (I) or the structure (II) to yield (RS)-N-ethyl-1-[phenyl]propan-2-amine substituted with R₁ on the phenyl ring or fenfluramine or an analog thereof occurs with ethylamine and preferably with a reducing agent chosen from the group consisting of alkaline cation or ammonium borohydride and alkaline cation or ammonium triacetoxyborohydride, in which the alkaline cation is different from lithium cation and mixtures thereof. Preferably, sodium borohydride or sodium triacetoxyborohydride or mixtures thereof is used as a reducing agent.

Preferably, the R₁ group in the ketone having the structure (I) or the structure (II) is CF₃, and therefore when the ketone having the structure (II) is used as a starting raw material the process leads to the formation of fenfluramine.

Preferably, the transformation of the ketone having the structure (I) or the structure (II) as above with ethylamine is performed in the presence of an organic reaction solvent.

This is done because the inventors of the present application have discovered unexpectedly that the transformation of the ketone having the structure (I) or of the ketone having the structure (II) is possible with highly favorable results by using sodium borohydride (or sodium triacetoxyborohydride or, less conveniently, sodium cyanoborohydride or a mixture of 2 or 3 reagents as above) as reducing agent, and if sodium borohydride or sodium triacetoxyborohydride (or a mixture of the two) is used, also obviating the problem of any residual cyanides. This invention is surprising because: while sodium borohydride was considered to be on the one hand a reducing agent that is not strong enough to be used in the reduction of double bonds like the bond of the enamine derivative that is formed by reaction of the ketone (3-trifluoromethyl phenylacetone) with ethylamine, where the double bond is conjugated with an aromatic ring in the presence of an electron-attractor group on the ring, on the other hand at the same time it is not selective enough to not reduce - separately of the imine/enamine double bond- also the ketone present in the reaction environment, yielding the corresponding hydroxyl impurity. While the sodium triacetoxyborohydride and the sodium cyanoborohydride were considered insufficiently powerful to reduce a conjugated enamine bond with an aromatic ring substituted with an electron-attractor group (see Journal of Organic chemistry Vol. 61 No. 11, 1996 p. 3849-3862 and ORGANIC PROCESS RESEARCH AND DEVELOPMENT Vol. 10 No. 5 2006 p. 971-1031, which are two very extensive reviews that summarize a large number of reactions conducted over time and which clearly show the lack of substrates substituted with electron-attractor groups). This finding is surprising, since sodium borohydride had been considered scarcely suitable for reductive aminations with respect to sodium cyanoborohydride, as shown by a monograph published in Organic Syntheses, Coll. Vol. 6, p. 499 (1988); Vol. 52, p. 124 (1972). As is known to the person skilled in the art, the journal Organic Synthesis is an encyclopedic collection of organic synthesis methods founded in 1921 and distributed freely on the web since 1998 (see: http://www.orgsyn.org). The journal is highly regarded in its field because it pays particular attention to the practical repeatability of the described syntheses, requiring every synthesis to be repeated successfully, prior to its publication, in a laboratory of at least one member of the editorial board who acts as a referee for the article submitted to him - and who then appears as a reviewer of the article next to the author.

From this standpoint, the suitability of sodium triacetoxyborohydride (or, as an alternative, of the less suitable sodium cyanoborohydride), found by the inventors of the present application, is unexpected, especially on the background of the intense and repeated efforts to modernize synthesis of fenfluramine over the last 50 years mentioned above.

The synthesis path that has been found and is claimed by the inventors of the present patent instead solves all the problems that have been identified and discussed in the preceding pages, since starting from an easily available raw material, trifluoromethyl phenylpropan-2-one (or analogs thereof), and by using safe and low-toxicity reagents it allows to obtain, with high yields and high purity, fenfluramine or isomers thereof and/or analogs thereof in a process of the 1 step/1 pot type or preferably of the 2 steps/1 pot type.

That the synthesis of fenfluramine or of an analog thereof was until today a considerable challenge is demonstrated also by "Fluorinated Heterocycles", published on January 1, 2009 and already mentioned above, in which the author John Welch, for the synthesis of a fenfluramine analog, chooses an inverted synthesis path, in which starting from pentafluorosulfanylbenzene in 5 steps he reaches an analog of fenfluramine substituted on the aromatic ring with an -SF₅ group instead of -CF₃. In practice, in this case, reductive amination with triacetoxyborohydride occurs between the 2-amino-1(3'-pentafluorosulfanylphenyl)propane and acetaldehyde. The inventor therefore avoids passing through an enamine intermediate conjugated with the electron-poor aromatic ring, which would have had unknowns in terms of feasibility, and develops a longer synthesis path and provides for the use of butyllithium at a temperature of -78°C, with a yield of the reductive amination step of 30% and with an overall yield of 7-8%. The choice of this "inverted" path therefore confirms the existence of severe prejudice against the synthesis undertaken instead by the inventors of the present application, since:
A) The enamine that is formed by reaction between trifluoromethyl phenylpropan-2-one is a particular enamine, in which the double enamine bond is conjugated with an electron-poor aromatic ring due to the presence of the trifluoromethyl group. This situation might have suggested that a particularly powerful reducing system would be necessary, such as a reduction with hydrogen under pressure with metallic catalysts, or a reduction by electrochemical methods with the mercury cathode or the like.
B) At the same time, it is known that the reaction between a ketone/aldehyde with a primary or secondary amine leads to an equilibrium among the 3 species: the initial ketone/aldehyde, the enamine and the imine. Equilibrium in this case, as noted earlier, is shifted toward the formation of enamine, but the reaction rate of the ketone to form the imine/enamine, as is known, is determined by the ΔE of the transition state. This parameter is not easily predictable and therefore on the basis of previous experience it was reasonable to assume that during this reduction the reduction product of ketone would also form in addition to fenfluramine in significant quantities. See for example "Organic Syntheses Coll. Vol. 6 p. 499 of 1988", wherein the yield in product of reductive amination with sodium borohydride is 3%.

In the synthesis found by the inventors, the ketone having the structure (II), which is initial material (i.e., 1-(3-trifluoromethyl)phenyl-propan-2-one), in which R₁ is CF₃, or an analog thereof in which R₁ is an electron-attractor group as stated explicitly in the present application) is commercially available from various sources or can be prepared according to various methods that are known in the literature (for example Org. Syntheses Coll., Vol II p. 391 and p. 487 o also US3198833).

In the synthesis found by the inventors, the term "electron-attractor group" must be interpreted and understood with a broad meaning, so as to comprise any chemical group or atom capable of inducing a delocalization of the enamine electron doublet on the adjacent aromatic ring. Therefore, the term "attractor group" comprises, but is not limited to, the following groups or atoms: halogen, -SF₅, carboxylic group, trihalogen methyl group, mesylate, tosylate, triflate, nitro, phosphate, sulfonic, amide and the like. Preferably, the term "electron-attractor group" is chosen from the group that consists of: CF₃, halogen, -SF₅, carboxylic group, trihalogen methyl group, different from CF₃, mesylate, tosylate, triflate, nitro, phosphate, sulfonic and amide.

The new process for preparing (RS)-N-ethyl-[phenyl]propan-2-amine substituted with R₁ on the phenyl ring, preferably fenfluramine (or analogs thereof), according to the present invention is performed preferably in a single reactor without intermediate isolation (procedure known as "one-pot"). According to a first embodiment of the new process, the first step consists preferably of the reaction between the ketone of the structure (I) or preferably, as exemplified below, of 1-(3-trifluoromethyl)phenyl-propan-2-one or an analog thereof, wherein R₁ is an electron-attractor group as above with ethylamine to yield to the intermediate "enamine":

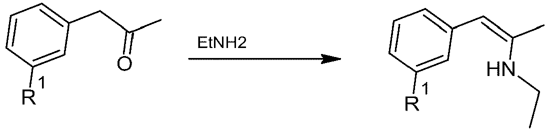

The second step, which occurs preferably without isolating the enamine intermediate, but simply by adding sodium borohydride or sodium triacetoxyborohydride (or less preferably sodium cyanoborohydride) (or mixtures of two or three of the latter) to the reaction environment, is indeed the reduction of the enamine intermediate (hereinafter exemplified for NaBH4, but equally possible for sodium triacetoxyborohydride or - less preferably - sodium cyanoborohydride):

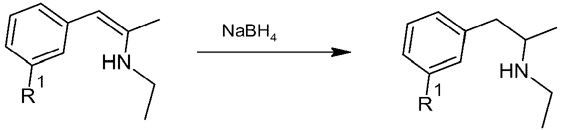

However, the inventors have discovered that is it is equally possible to perform the synthesis in a 1 step/1 pot format, i.e., by joining simultaneously the ketone with the structure (I) or preferably 1-(3-trifluoromethyl)phenyl-propan-2-one or an analog thereof, in which R₁ is an electron-attractor group as above, ethylamine (or a source thereof) and the reducing agent considered by the present invention.

It has been discovered that the reaction described here can, in all of its embodiments, be performed in relatively bland conditions.

The procedure for forming the enamine identified in the present invention provides for the reaction between the ketone having the structure (I) or preferably having the structure (II) and ethylamine at a suitable temperature and at atmospheric pressure, preferably in the presence of the organic reaction solvent. The ethylamine source can be, by way of example but not limited thereto, base ethylamine or an ethylamine salt, such as ethylamine hydrochloride or ethylamine hydrobromide or the like. The ethylamine source can be added to the reaction as such or in a mixture with a suitable vehicle, such as for example an organic solvent (for example but not necessarily the same organic reaction solvent) or water.

In the synthesis found by the inventors, the organic reaction solvent can be any suitable organic solvent. Preferably, the reaction solvent can be an alcohol, an ether such as for example tetrahydrofuran or an ester, such as for example ethyl acetate, or can be an aprotic dipolar solvent, such as for example acetonitrile or dimethylformamide, or can be a halogenated solvent, particularly a chlorinated one, such as dichloroethane or dichloromethane or chloroform, or a halogenated solvent further substituted with a different functional group, such as for example trifluoroethanol, or a hydrocarbon, preferably an aromatic one, such as toluene. Among the above cited, alcohols, ethers and esters are preferred; among the latter, instead, alcohols and ethers are particularly preferred. Among alcohols, preference is given in particular to monoalcohols with a linear or ramified chain and bis-alcohols, for example ethylene glycol and other linear or ramified chain alcohols with 2 hydroxy groups. Among ethers, preference is given to mono ethers or bis ethers with a linear or ramified chain or cyclic ethers, such as for example THF. Even more preferably, the suitable reaction solvent is a linear or ramified monoalcohol with a number of carbon atoms between 1 and 6, such as for example methanol or ethanol or propanol or butanol or isopropanol. Methanol is particularly preferred.

The inventors have found preferred experimental conditions in which the formation of the enamine in the first embodiment described herein can occur advantageously. In fact, it has been discovered unexpectedly and surprisingly that the formation of enamine can occur very rapidly, with the consequent advantage both of the selectivity of the enamine bond reduction reaction with respect to the ketone bond and for the productivity of the synthesis process described herein.

The following Table **A** provides a first overview of the conducted explorations (tests). All the tests listed in Table **A** were conducted according to the conditions of example 1 given hereinafter in the experimental section; the parameters that were changed with respect to said example 1 are given in the table (tests 1, 2, 4, 5-10). It should be noted instead that test 3 (recited in each of the three quadrants of Table **A** below) is example 1 itself. Unless otherwise specified in the present application, all temperatures and pressures refer to ambient values.

**TABLE A**

| **Test** | Eq. EtNH₂ **hydrochloride** | Enamination temperature | Reaction time (enamination) | Yield^{a} **fenfluramine** | Purity **fenfluramine** (HPLC)^{b} |
|---|---|---|---|---|---|
| 1 | 1.0 | 20°C | 4.5h | 46% | 52% |
| 2 | 2.0 | 20°C | 4.5h | 71% | 72% |
| **3** | 3.5 | 20°C | 4.5h | 72% | 77% |
| 4 | 10.0 | 20°C | 4.5h | 71% | 82% |
| 5 | 3.5 | 0°C | 4.5h | 67% | 84% |
| **3** | 3.5 | 20°C | 4.5h | 72% | 77% |
| 6 | 3.5 | 40°C | 4.5h | 70% | 72% |
| 7 | 3.5 | 70°C | 4.5h | 42% | 49% |
| 8 | 3.5 | 20°C | 0.5h | 74% | 84% |
| **3** | 3.5 | 20°C | 4.5h | 72% | 77% |
| 9 | 3.5 | 20°C | 24h | 71% | 79% |
| 10 | 3.5 | 20°C | 5 min | 76% | 78% |

| | | | | | |
|---|---|---|---|---|---|
| ^{a.} This is a normalized yield, i.e., calculated by multiplying the yield of free base crude fenfluramine by the HPLC purity value (i.e., the percentage area of the fenfluramine peak in the chromatogram). ^{b.} Purity evaluated at 210 nM | | | | | |

Analysis of the table first of all shows that the (normalized) yields obtained are always at least 40%, preferably at least 70%, proving the fact that sodium borohydride is an excellent reagent for synthesizing fenfluramine or analogs thereof with electron-attractor groups starting from the ketone having the structure (II). A further optimization can be obtained by selecting appropriately the number of equivalents of ethylamine used in the reaction, and the reaction temperature during the formation of the enamine, while - surprisingly - the influence of the enamine formation time is almost nonexistent, indicating therefore that it forms substantially instantaneously.

It should in fact be noted that the conditions studied in table **A** are based on example 1 and therefore do not provide for particular measures aimed at removing the water that forms during enamination, such as can be for example the use of dehydrating agents (such as for example anhydrous magnesium sulfate, anhydrous sodium sulfate and other similar agents or zeolites, activated molecular sieves and the like, the use of a suitable solvent such as toluene or benzene, in continuous azeotropic removal of the water that forms during the reaction (Dean-Stark apparatus), or the addition of Lewis acids such as for example titanium compounds such as Ti(IV) isopropoxide and the like). Although theoretically the use of methods for removing the water as above is not excluded, it is evident that their use is not indispensable.

As regards the ethylamine equivalents, it is preferred to use amine in excess, using at least 1.25, preferably 1.5 - 15 equivalents.

As regards the reaction temperature during enamine formation, working in a range between -10°C and 50°C is preferred.

According to a preferred embodiment of the new process described herein, once the ketone having the structure (I) or preferably the 1-(3-trifluoromethyl)phenyl-propan-2-one or an analog thereof as described herein has been converted into the enamine, the enamine intermediate, preferably is not isolated but, directly in the same reactor, the reduction step is performed by adding sodium borohydride, sodium triacetoxyborohydride, or less preferably sodium cyanoborohydride, or a mixture of two or three of the antecedents.

The reduction step can be performed in bland conditions. For example, this step can occur at atmospheric pressure and at temperatures in the range from -20°C in reflux of the reaction solvent. Preferably, reduction occurs in a temperature interval from -10°C to 50°C. Preferably, the reduction can occur at temperatures from 0°C to 30°C. More preferably, the reduction is performed at 18°C to 25°C.

More generally, it is possible to conclude that the invention as described herein allows, for example by using a slight amine excess (for example at least 2 equivalents) and by conducting the reduction for example in a temperature interval from 20°C to 40°C, to obtain crude free base fenfluramine with a normalized yield, i.e., calculated by multiplying the free base crude fenfluramine yield by the HPLC purity value (i.e., the percentage area of the fenfluramine peak in the chromatogram), of at least 70%, and with an HPLC purity at 210 nm of at least 70%, regardless of the enamination time.

Further tests conducted by varying the nature of R₁, the position of R₁ on the phenyl ring and/or the reducing agent according to the present invention are instead given in the following Table **B**. Here, too, the conducted tests follow the approach of example 1 as described hereinafter in the experimental section, varying the parameters given in Table **B**. In the case of test 13, the initial ketone having the structure (II), dissolved in THF, the ethylamine solution and the reducing agent were drip-fed simultaneously into the reaction vessel.

**TABLE B**

| Test | Ketone (R₁) | Solvent | Eq. EtNH₂ | Reducing agent | Enamination temperature | Reaction time (enamination) | Yield^{a} | Purity (GC) |
|---|---|---|---|---|---|---|---|---|
| 11 | 3-F | MeOH | 2.64 | NaBH₄ | 20°C | 15 min | 78% | 93% |
| 12 | 4-F | MeOH | 2.64 | NaBH₄ | 20°C | 15 min | 75% | 96% |
| 13 | 3-CF₃ | THF | 1.5 | NaBH(OAc)₃ | 20°C | 0 min | 71% | 88% |
| 14 | 3-CF₃ | MeOH | 1.13 | NaBH₃CN | 20°C | 15 min | 60% | 72%^{b} |

| | | | | | | | | |
|---|---|---|---|---|---|---|---|---|
| ^{a.} In this case also, this is a normalized yield, calculated by multiplying the yield of free base crude fenfluramine (or isomer/analog thereof) by the purity value (percentage area of the peak of fenfluramine or of its isomer/analog at the chromatogram), this time at the GC. ^{b.} Despite a GC purity of 72% of the free base obtained from the synthesis of fenfluramine by reduction with sodium cyanoborohydride, mere conversion to hydrochloride according to the first part of the procedure of example 2a of this patent has led, without distillation of the free base and without recrystallization from 2-butanol, to a crude product with a melting point already of 170°C (see the DSC chart attached as **Figure 2**). | | | | | | | | |

Table **B** therefore lists four examples in which, in tests 11 and 12, synthesis on substrates was performed (ketones having the structure (II) and having the structure (I), respectively) with a different electron-attractor group on the aromatic ring, specifically 3-fluorophenylpropan-2-one and 4-fluorophenylpropan-2-one. As can be seen from the data given in Table **B**, the results are substantially equivalent to those in which the electron-attractor group is CF₃.

In tests 13 and 14, synthesis was instead performed on the ketone having the structure (II) provided with the 3-trifluoromethyl group on the aromatic ring by using different reducing agents (sodium triacetoxyborohydride and sodium cyanoborohydride). As can be seen, the results are substantially comparable to those of sodium borohydride of the preceding Table **A**.

As mentioned above, in the ketone having the structure (I) or having the structure (II), R₁ is -CF₃ or another electron-attractor group cited above. Preferably, R₁ is CF₃. If another leaving group R₂ other than the group R₁ is instead present in the initial material, the process provides for a previous step in which said leaving group R₂ is substituted with -CF₃ (or with another electron-attractor group as above), thus converting the different leaving group R₂ into the group R₁ as defined in the present application. This transformation can be performed by using methods known to persons skilled in the art.

A preparation of (RS)-N-ethyl-[phenyl]propan-2-amine substituted with R₁ on the phenyl ring or of fenfluramine (or of an analog thereof) obtained with the process according to the present invention is characterized already by a good degree of chemical purity. However, to further purify the active ingredient, a step of distillation of free base (RS)-N-ethyl-[phenyl]propan-2-amine substituted with R₁ on the phenyl ring or of free base fenfluramine (or of its analog) can be performed. Preferably, this distillation is a distillation at reduced pressure.

If the formation of a salt of (RS)-N-ethyl-[phenyl]propan-2-amine substituted with R₁ on the phenyl ring or of fenfluramine (or of an analog thereof) is desired, the process can include additionally a salification step.

According to the present invention, the purification of free base (RS)-N-ethyl-[phenyl]propan-2-amine substituted with R₁ on the phenyl ring or of free base fenfluramine obtained as described here (or of an analog thereof) can be performed by distilling the free base and/or by crystallizing a salt of (RS)-N-ethyl-[phenyl]propan-2-amine substituted with R₁ on the phenyl ring or of fenfluramine (or of an analog thereof). Depending on the desired degree of purity, both purification processes are performed in sequence (first distillation and then crystallization), or only one of the two purification processes is performed.

For example, if it is necessary to prepare a halide salt of fenfluramine (for example fenfluramine hydrochloride), this can be done by treating fenfluramine obtained with the process according to the present invention with a suitable hydrohalic acid (for example hydrochloric acid). Likewise, it is possible to prepare other pharmaceutically acceptable fenfluramine salts, such as for example sulfate, nitrate, hydrobromide, hydriodate and salts of fenfluramine with carboxylic and sulfonic, aliphatic, alicyclic, aromatic and heteroaromatic acids, such as citrate, tartrate, dibenzoyltartrate, formiate, acetate, 4-chlorobenzene acetate, propionate, oxalate, succinate, glycolate, lactate, maleate, fumarate, camphorate, camphorsulfonate, benzene sulfonate, trifluoromethane sulfonate, benzoate, alginate, etc. What has been stated above applies also to (RS)-N-ethyl-[phenyl]propan-2-amine substituted with R₁ on the phenyl ring and to the analogs of fenfluramine obtained according to the present invention.

Additionally, the fenfluramine salt thus obtained can be purified further to yield an active ingredient with very high purity, for example by trituration or (re)crystallization from an appropriate organic solvent or mixtures of organic solvents.

Preferably, the fenfluramine salt is the hydrochloride and it is (re)crystallized by an appropriate organic solvent. Preferably, the organic solvent is a ketone or an alcohol or an ester or an ether or mixtures thereof. More preferably, the (re)crystallization solvent is one among isopropanol, iso-butanol, tert-butanol, sec-butanol, methyl tert-butyl ketone, methyl isobutyl ketone, acetone, ethyl acetate. What has been stated above applies equally for salts of (RS)-N-ethyl-[phenyl]propan-2-amine substituted with R₁ on the phenyl ring and for salts of the analogues of fenfluramine obtained according to the present invention.

Therefore, as is evident from what has been discussed so far, the process of the present invention can be used to prepare (RS)-N-ethyl-[phenyl]propan-2-amine substituted with R₁ on the phenyl ring, preferably fenfluramine or analogs thereof and salts thereof, in an easy manner, and the main advantages with respect to the methods that are present in the literature are listed hereafter:
- the process is short and occurs in a single reactor by means of one or at most two synthesis steps, without isolating intermediates. The fact that the process is so simple and does not provide for multiple steps of synthesis, isolation, purification has the effect that a smaller number of impurities is generated with respect to long processes, with multiple steps. Fenfluramine with a high degree of purity is therefore obtained. Another benefit of such a short process is that the cost of the active ingredient fenfluramine is lower and the process is economic and advantageous.
- The process does not use hydrogen gas and pressurized reactors but occurs at atmospheric pressure and is a safe process.
- The process does not use transition metals (such as nickel, palladium, platinum and others, used for example as catalysts) and/or reagents based on heavy metals (such as mercury and others) that can contaminate the final active ingredient. One therefore obtains fenfluramine, which does not contain potentially noxious metals and is suitable also for administration in a chronic therapy for children.
- The process does not use toxic reagents, in particular, according to a preferred embodiment of the present invention, it does not use cyanides.
- The process does not use highly dangerous or explosive reagents, such as the ones used in other processes described in the prior art, for example azides (for example sodium azide), epoxides, Grignard reagents or lithium aluminum hydride.

Furthermore, the process according to the present invention is also preferable with respect to methods that are present in the existing literature, since fenfluramine is obtained with high yields, i.e., the (normalized) yields obtained can be at least 40%, preferably at least 70%, and this with a degree of purity (HPLC and/or GC) that is high (for example at least 70%) and with a better impurity profile.
- Therefore, according to a further aspect of the present invention, with the process described in the present invention it is possible to obtain preparations of (RS)-N-ethyl-[phenyl]propan-2-amine substituted with R₁ on the phenyl ring or of fenfluramine (or analogs thereof) or of a pharmaceutically acceptable salt thereof with the following purity characteristics: individual impurity levels lower than 0.2%, preferably lower than 0.1%, assessed on the basis of HPLC analyses and expressed as a percentage area of the peak of impurity in the chromatogram.
   - According to a further aspect of the present invention, with the process described in the present invention it is possible to obtain preparations of (RS)-N-ethyl-[phenyl]propan-2-amine substituted with R₁ on the phenyl ring or of fenfluramine (or analogs thereof) or of a pharmaceutically acceptable salt thereof with the following purity characteristics: content of heavy metals or transition metals: lower for the individual metal than 2 ppm (by weight), preferably lower than 1 ppm (by weight).

The fenfluramine preparations as above, obtainable according to the method of the present invention, can be used, after the addition of suitable pharmaceutically acceptable excipients, to obtain new pharmaceutical compositions of fenfluramine, characterized by the high purity indicated above. The same applies for preparations of (RS)-N-ethyl-[phenyl]propan-2-amine substituted with R₁ on the phenyl ring or for analogues of fenfluramine obtainable according to the method of the present invention.

### EXAMPLES

The present invention is exemplified by, but not limited to, the following examples:

### Example 1 - Synthesis of fenfluramine

A suspension of sodium hydroxide (34.62 g - 0.866 mol, 3.5 eq) in 170 mL of methanol, under mechanical agitation, receives the addition, drop by drop, over the course of 30 minutes, of a solution of ethylamine hydrochloride (70.59 g - 0.866 mol, 3.5 eq) in 165 mL of methanol, followed by 1-(3-trifluoromethyl)phenyl-propan-2-one (50 g - 0.247 mol). The mixture is left under agitation at 20°C for 4.5 hours, then cooling to 0°C is performed and a solution of sodium borohydride (9.36 g - 0.247 mol) in 19 mL of sodium hydroxide 1M in water is then added drop by drop, keeping the temperature below 10°C. The reaction is then left under agitation at 20°C for another 2 hours. Once the reaction is complete, 270 mL of methanol are removed at a reduced pressure at 40°C and then 200 mL of water are added and the mixture is extracted with heptane (200 mL). The aqueous phase is eliminated and the organic phase is washed with water (200 mL x 3). The organic phase is concentrated at 50°C at reduced pressure to yield free base fenfluramine as colorless oil. Yield: 72%; purity: 77% - as listed in test 3 of table **A** above.

### Example 2 - Purification of fenfluramine

Purification of free base fenfluramine can be performed in two ways:
- distillation of the free base
- crystallization of the fenfluramine hydrochloride salt

Depending on the degree of purity that is desired, both purification processes are performed in sequence (distillation first and then crystallization), or only one of the two purification processes is performed.

### Example 2a - Distillation:

Free base fenfluramine (10 g), prepared as in Example 1, is distilled under reduced pressure with a distillation column of the Vigreux type: the distillation heads are eliminated, the fraction that is distilled at 89-90°C at 6 mmHg, which is the active ingredient fenfluramine (8.5 g) with a high degree of purity, is collected.

### Example 2b - Conversion into hydrochloride salt and crystallization:

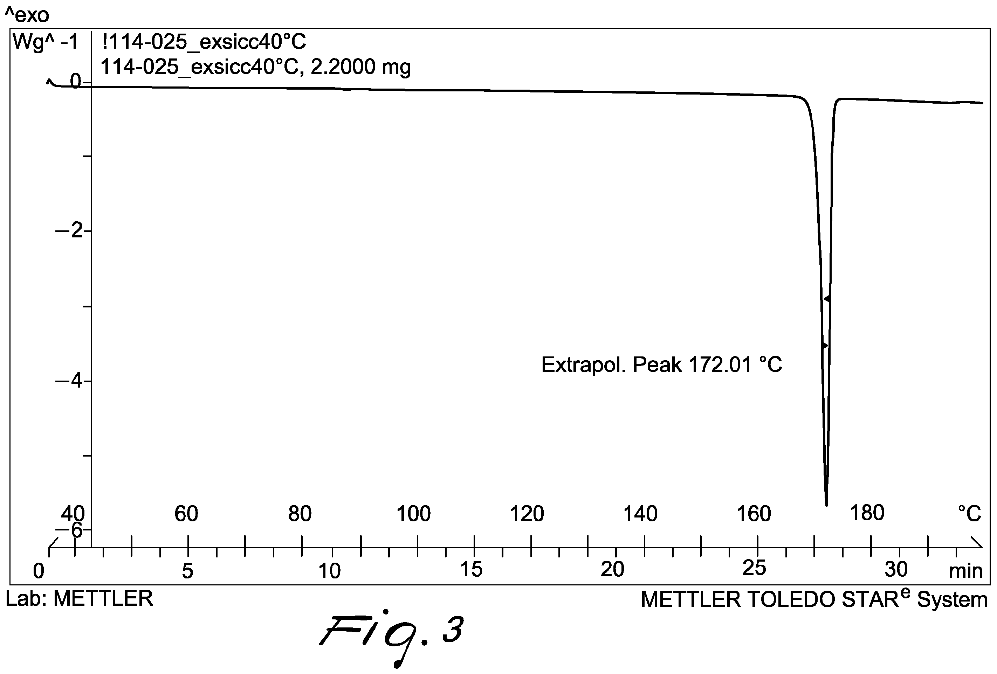

Crude fenfluramine, prepared as in Example 1, or purified fenfluramine as in Example 2a, is dissolved in 125 mL of ethyl acetate, and cooling is performed to 0°Celsius under agitation. 272 mL of a solution of 1M HCl in ethyl acetate are added drop by drop at 0°C. The precipitate that forms is filtered and washed with ethyl acetate (125 mL x 2) to yield approximately 55 g of solid fraction. The solid fraction is crystallized by 2-butanol (260 mL), keeping the solid for 22 hours at 3°C under slow agitation before filtering it. Filtering is performed and washing is performed with cold 2-butanol. The solid fraction, fenfluramine hydrochloride, is dried in a vacuum stove, yielding 51.7 g of product. A DSC of the resulting product is shown in Figure 3.

The present invention is further defined by the following items:
1. A method for synthesis of (RS)-N-ethyl-[phenyl]propan-2-amine substituted with R₁ on the phenyl ring or of a pharmaceutically acceptable salt thereof, comprising the transformation of a ketone having the structure (I): 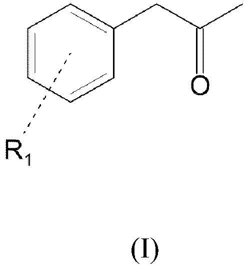
   wherein R₁ is an electron-attractor group chosen in turn from the group that consists of CF₃, halogen, -SF₅, carboxyl group, trihalogen methyl group other than CF₃, mesylate, tosylate, triflate, nitro, phosphate, sulfonate and amide,
   with ethylamine
   and with a reducing agent chosen from the group consisting of alkaline cation or ammonium borohydride, alkaline cation or ammonium triacetoxyborohydride and alkaline cation or ammonium cyanoborohydride, in which the alkaline cation is always different from lithium cation and mixtures thereof, to yield (RS)-N-ethyl-[phenyl]propan-2-amine substituted with R₁ on the phenyl ring, optionally followed by the transformation of the obtained (RS)-N-ethyl-[phenyl]propan-2-amine substituted with R₁ on the phenyl ring into a pharmaceutically acceptable salt.
2. The method according to item 1 for preparing fenfluramine, an analog thereof as indicated hereafter, or a pharmaceutically acceptable salt thereof, comprising the transformation of a ketone having the structure (II): 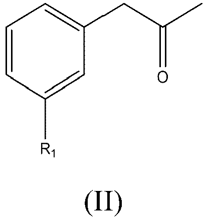
   wherein R₁ is CF₃ or wherein R₁ is an electron-attractor group chosen in turn from the group consisting of halogen, -SF₅, carboxyl group, trihalogen methyl group other than CF₃, mesylate, tosylate, triflate, nitro, phosphate, sulfonate and amide,
   with ethylamine
   and with a reducing agent chosen from the group consisting of sodium borohydride, sodium triacetoxyborohydride and sodium cyanoborohydride and mixtures thereof, to yield fenfluramine or an analog thereof in which R₁ is an electron-attractor group as above, optionally followed by the transformation of the obtained fenfluramine or of an analog thereof as above into a pharmaceutically acceptable salt.
3. The method according to item 1 or 2, wherein the transformation of the ketone having the structure (I) or (II) occurs with ethylamine and with a reducing agent chosen from the group that consists of sodium borohydride and sodium triacetoxyborohydride and mixtures thereof.
4. The method according to one of items 1-3, wherein the group R₁ in the ketone having the structure (I) or the structure (II) is CF₃.
5. The method according to one of items 1-4, wherein the transformation of the ketone having the structure (I) or the structure (II) is performed in the presence of at least one organic reaction solvent.
6. The method according to item 5, wherein the organic reaction solvent is a linear or ramified alcohol with a number of carbon atoms from 1 to 6, or is a cyclic ether with 3-5 carbon atoms.
7. The method according to item 6, wherein the reaction solvent is methanol or is THF.
8. The method according to one of items 1-7, wherein the reducing agent is added to the reaction mixture obtained from the mixing of ketone having the structure (I) or the structure (II) and ethylamine.
9. The method according to one of items 1-8, in which at least 1.25 equivalents of ethylamine are used with respect to the ketone having the structure (I) or the structure (II).
10. The method according to one of items 8 or 9, wherein the reaction mixture obtained from the mixing of ketone having the structure (I) or the structure (II) and ethylamine is made to react for at least 5 minutes in a temperature interval from -10°C to 50°C before adding the reducing agent.
11. The method according to items 10, wherein, after the addition of the reducing agent, the temperature is maintained in a temperature interval from -10°C to 50°C.
12. The method according to one or more of items 1-11, furthermore comprising a step of salification and/or distillation of the base (RS)-N-ethyl-[phenyl]propan-2-amine substituted with R₁ on the phenyl ring or of the base fenfluramine or of the analog thereof that has been obtained.
13. The method according to item 12, wherein the salification step consists in causing (RS)-N-ethyl-[phenyl]propan-2-amine substituted with R₁ on the phenyl ring or fenfluramine or analog thereof to come into contact with hydrochloric acid to yield hydrochloride of (RS)-N-ethyl-[phenyl]propan-2-amine substituted with R₁ on the phenyl ring or of fenfluramine or of an analog thereof.
14. The method according to one of items 1-13, wherein the (RS)-N-ethyl-[phenyl]propan-2-amine substituted with R₁ on the phenyl ring or fenfluramine or analog thereof are obtained from the ketone having the structure (I) or the structure (II) without using benzene, toluene, carbon tetrachloride, 1,2-dichloroethane, 1,1-dichloroethane and 1,1,1-trichloroethane, azides, epoxides, heavy metals and/or transition metals, lithium aluminum hydride and Grignard reagents.
15. A preparation of (RS)-N-ethyl-[phenyl]propan-2-amine substituted with R₁ on the phenyl ring, wherein R₁ is an electron-attractor group chosen in turn from the group consisting of CF₃, halogen, -SF₅, carboxyl group, trihalogen methyl group other than CF₃, mesylate, tosylate, triflate, nitro, phosphate, sulfonate and amide,
   or of a pharmaceutically acceptable salt thereof, with a purity as follows:
   - levels of individual impurities, assessed by HPLC analysis, lower than: 0.2% (area) each, preferably lower than 0.1% (area) each,
      and/or
   - content of heavy metals or transition metals: lower for the individual metal than 2 ppm (by weight), preferably lower than 1 ppm (by weight).
16. The preparation according to item 15, wherein the (RS)-N-ethyl-[phenyl]propan-2-amine substituted with R₁ on the phenyl ring is fenfluramine or an analog thereof in which instead of the CF₃ group there is another electron-attractor group chosen in turn from the group that consists of halogen, -SF₅, carboxyl group, trihalogen methyl group other than CF₃, mesylate, tosylate, triflate, nitro, phosphate, sulfonate and amide.
17. The preparation according to item 15 or 16, obtainable according to the process of one of items 1-14.
18. A pharmaceutical composition, comprising the preparation according to one of items 15-17 and at least one pharmaceutically acceptable excipient.

## Claims

1. A method for synthesis of fenfluramine or of a pharmaceutically acceptable salt thereof, comprising the transformation of a ketone having the structure (II): 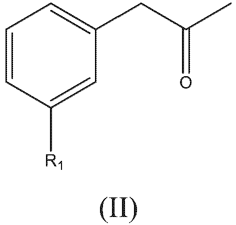
wherein R₁ is CF₃,
with ethylamine
and with a reducing agent that is alkaline cation or ammonium triacetoxyborohydride, in which the alkaline cation is always different from lithium cation, to yield fenfluramine, optionally followed by the transformation of the obtained fenfluramine into a pharmaceutically acceptable salt.

2. The method according to claim 1, wherein the transformation of the ketone having the structure (II) occurs with ethylamine and with a reducing agent that is sodium triacetoxyborohydride.

3. The method according to claim 1 or 2, wherein the transformation of the ketone having the structure (II) is performed in the presence of at least one organic reaction solvent.

4. The method according to claim 3, wherein the organic reaction solvent is a linear or ramified alcohol with a number of carbon atoms from 1 to 6, or is a cyclic ether with 3-5 carbon atoms.

5. The method according to claim 4, wherein the reaction solvent is methanol or is THF.

6. The method according to one of claims 1-5, wherein the reducing agent is added to the reaction mixture obtained from the mixing of ketone having the structure (II) and ethylamine.

7. The method according to one of claims 1-6, in which at least 1.25 equivalents of ethylamine are used with respect to the ketone having the structure (II).

8. The method according to claim 6 or 7, wherein the reaction mixture obtained from the mixing of ketone having the structure (II) and ethylamine is made to react for at least 5 minutes in a temperature interval from -10°C to 50°C before adding the reducing agent.

9. The method according to claim 8, wherein, after the addition of the reducing agent, the temperature is maintained in a temperature interval from -10°C to 50°C.

10. The method according to one or more of claims 1-9, furthermore comprising a step of salification and/or distillation of the base fenfluramine that has been obtained.

11. The method according to claim 10, wherein the salification step consists in causing fenfluramine to come into contact with hydrochloric acid to yield hydrochloride of fenfluramine.

12. The method according to one of claims 1-11, wherein the fenfluramine is obtained from the ketone having the structure (II) without using benzene, toluene, carbon tetrachloride, 1,2-dichloroethane, 1,1-dichloroethane and 1,1,1-trichloroethane, azides, epoxides, heavy metals and/or transition metals, lithium aluminum hydride and Grignard reagents.

13. A preparation of fenfluramine or of a pharmaceutically acceptable salt thereof obtainable according to the process of one of claims 1-12, with a purity as follows:
- levels of individual impurities, assessed by HPLC analysis, lower than: 0.2% (area) each, preferably lower than 0.1% (area) each,
and/or
- content of heavy metals or transition metals: lower for the individual metal than 2 ppm (by weight), preferably lower than 1 ppm (by weight).

14. A pharmaceutical composition, comprising the preparation according to claim 13 and at least one pharmaceutically acceptable excipient.

15. The preparation of fenfluramine or of a pharmaceutically acceptable salt thereof according to claim 13 or the pharmaceutical composition according to claim 14 for use in a chronic therapy for the treatment of the symptoms of epilepsy and other correlated severe disorders, preferably wherein the chronic therapy is for children.
